**Europäisches Patentamt**

**European Patent Office**

**Office européen des brevets**

(19)

(11) Veröffentlichungsnummer: **0 042 928**
**B1**

(12) # EUROPÄISCHE PATENTSCHRIFT

(45) Veröffentlichungstag der Patentschrift:
**18.07.84**

(21) Anmeldenummer: **81103118.6**

(22) Anmeldetag: **25.04.81**

(51) Int. Cl.³: **C 07 C 67/38, B 01 J 23/94**

(54) **Verfahren zur Herstellung von Fettsäureestern.**

(30) Priorität: **25.06.80 DE 3023765**

(43) Veröffentlichungstag der Anmeldung:
**06.01.82 Patentblatt 82/1**

(45) Bekanntmachung des Hinweises auf die Patenterteilung:
**18.07.84 Patentblatt 84/29**

(84) Benannte Vertragsstaaten:
**AT BE FR GB IT NL**

(56) Entgegenhaltungen:
**EP - A - 0 001 242**
**EP - A - 0 008 024**
**EP - A - 0 017 051**
**DE - A - 2 713 195**
**DE - A - 2 836 518**
**US - A - 4 041 057**

(73) Patentinhaber: **CHEMISCHE WERKE HÜLS AG,**
**Postfach 1320, D-4370 Marl 1 (DE)**

(72) Erfinder: **Müller, Wolfgang Hans Eduard, Dr., Bitterfelder**
**Strasse 9a, D-4370 Marl (DE)**
Erfinder: **Hofmann, Peter, Dr., Lipper Weg 193,**
**D-4370 Marl (DE)**

## Beschreibung

Es ist bekannt, daß man durch Umsetzung von Olefinen mit Kohlenmonoxid und H-aciden Komponenten, wie z. B. Alkanolen, in Gegenwart eines Katalysators, der ein Metall der 8. Nebengruppe des Periodischen Systems der Elemente und gegebenenfalls einen Promotor enthält, Fettsäureester herstellen kann (J. Falbe, Synthesen mit Kohlenmonoxid, Springer-Verlag, Berlin, Heidelberg, New York, 1967).

Eine bevorzugte Variante dieser als Hydrocarboxylierung bezeichneten Reaktion ist die Umsetzung in Gegenwart von kobalthaltigen Katalysatoren. Als besonders bevorzugte Ausführungsform hat sich der zusätzliche Einsatz von Pyridin oder einem nichtorthosubstituierten Alkylpyridin als Promotor erwiesen.

Ein wesentliches Problem dieser homogen katalysierten Reaktion ist die verlustfreie und technisch einfach zu praktizierende Rückgewinnung des relativ teuren Kobalts aus dem Reaktionsgemisch in einer Form, die seinen Wiedereinsatz als Katalysator gestattet.

Nach den in der DE-OS 1 963 804 und der US-PS 3 507 891 beschriebenen Verfahren kann diese Aufgabe durch fraktionierte Destillation des durch Hydrocarboxylierung erhaltenen Reaktionsgemisches gelöst werden, wobei das als Katalysator eingesetzte Kobalt zusammen mit dem Destillationssumpf anfällt.

Eine solche Verfahrensweise führt jedoch bei wiederholter bzw. kontinuierlicher Anwendung gemäß den Ausführungen in der DE-PS 921 988 und der EP-OS 0 008 024 zu einer Anreicherung hochsiedender organischer Verunreinigungen im Destillationssumpf. Die Menge des Rückstandes nimmt dadurch ständig zu, so daß er von Zeit zu Zeit vollständig aufgearbeitet werden muß.

Eine Aufarbeitung des Destillationssumpfes durch Auskochen mit einer Carbonsäure oder Wasser, wie sie in DE-PS 921 988 vorgeschlagen wird, bereitet aufgrund der zähen und klebrigen Konsistenz derartiger Rückstände verfahrenstechnische Schwierigkeiten, ist im Falle der Anwendung von Carbonsäuren mit Korrosionsproblemen belastet und zwingt beim Auskochen mit Wasser aus Umweltgründen zu einer vollständigen Aufarbeitung der Wasserphase.

Die Extraktion des Destillationssumpfes mit $CO_2$, $C_2-C_4$-Paraffin, $C_2-C_4$-Olefin oder einem Halogenkohlenwasserstoff oberhalb der kritischen Temperatur und des kritischen Druckes dieser Verbindungen, wie sie in EP-OS 0 008 024 vorgeschlagen wird, ist infolge der anzuwendenden Drucke von über 100 bar keine einfach zu lösende Aufgabe. Zudem ist eine solche Verfahrensweise durch die Risiken, die sich aus dem Umgang mit leicht flüchtigen und leicht entzündbaren Verbindungen (niedere Olefine und Paraffine) ergeben, belastet. Bei einer Extraktion mit Halogenkohlenwasserstoffen, wie z. B. $CClF_3$, besteht zudem die Gefahr der Katalysatorvergiftung durch Einschleppen von Halogenverbindungen in die Hydrocarboxylierungsreaktion.

Dem Zwang zur Aufarbeitung des Destillationssumpfes entgeht man, wenn man das in DE-PS 2 159 139 beschriebene Verfahren zur Katalysatorrückgewinnung anwendet. Die Hydrocarboxylierungsreaktion wird nach diesem Verfahren in Gegenwart eines Methanolüberschusses als Veresterungskomponente und unter Zugabe von Kohlenwasserstoff zum ausreagierten Reaktionsgemisch durchgeführt. Es kommt so zur Ausbildung zweier Phasen, von denen die untere mindestens 95 Gewichtsprozent des als Katalysator eingesetzten Kobalts enthält. Diese Phase kann dann direkt in die Hydrocarboxylierungsreaktion zurückgeführt werden.

Die Nachteile dieses Verfahrens sind einmal die Beschränkung des Verfahrens auf die Herstellung von Methylestern und zum anderen der Verlust des in der oberen Phase verbliebenen Kobalts (bis zu 5 Gewichtsprozent).

Nach dem Verfahren der US-PS 4 041 057 erreicht man eine vollständige Kobaltrückgewinnung dadurch, daß man das gemäß dem Verfahren nach DE-PS 2 159 139 in der oberen Phase verbliebene Kobalt als Destillationssumpf zurückgewinnt und den so erhaltenen teerartigen Rückstand bei Temperaturen von 1000 bis 4000°F verbrennt, das hierbei gebildete Kobaltoxid aus den Verbrennungsgasen herausfiltert und durch Umsetzung mit Kohlenmonoxid und Wasserstoff in das Kobaltcarbonyl überführt. Letzteres kann wieder in die Hydrocarboxylierung zurückgeführt werden.

Bei einer Kombination des Verfahrens nach der DE-PS 2 159 139 und der US-PS 4 041 057 ist zwar eine vollständige Rückgewinnung des Kobalts möglich, die Rentabilität des Gesamtverfahrens wird jedoch durch den Einsatz großer Kohlenwasserstoffmengen, die die destillative Aufarbeitung erheblich belasten, sowie durch eine aufwendige, mehrstufige Rückgewinnung des in der oberen Phase verbliebenen Restkobalts beeinträchtigt.

Aufgabe der vorliegenden Erfindung war es daher, ein allgemein anwendbares und technisch in einfacher und wirtschaftlicher Weise durchführbares Verfahren zur verlustfreien Rückgewinnung von bei der Hydrocarboxylierung eingesetzter Kobaltkatalysatoren zu entwickeln.

Diese wurde überraschenderweise durch die in den Patentansprüchen beschriebenen Maßnahmen gelöst. Überraschend deshalb, weil es angesichts der Ausführungen in der DE-PS 1 963 804 und der EP-OS 0 008 024 nicht zu erwarten war, daß durch die in den Ansprüchen beschriebene spezielle Form der destillativen Aufarbeitung, eine Anreicherung von Hochsiedern in dem hierbei anfallenden kobalthaltigen Destillationssumpf vermieden und damit auf seine Aufarbeitung zur Entfernung von Hochsiedern verzichtet werden kann.

**0 042 928**

Das erfindungsgemäße Verfahren kann im Prinzip bei allen Hydrocarboxylierungsverfahren zur Herstellung von Fettsäureestern angewandt werden, bei denen ein Katalysator, bestehend aus einer Kobaltverbindung und Pyridin und/oder einem nichtorthosubstituierten Alkylpyridin, eingesetzt wird (z. B. Verfahren gemäß US-PS 3 507 891 und deutscher Patentanmeldung P 29 12 489.8, entsprechend EP-A1-17 051). So ist vor allem die Wahl des eingesetzten Olefins unkritisch, d. h., es können sowohl geradkettige oder verzweigte $\alpha$-Olefine als auch Olefine mit innenständiger Doppelbindung eingesetzt werden. Aber auch Olefine mit mehr als einer Doppelbindung und solche mit Substituenten, wie z. B. Aryl-, Cyano-, Carboximethyl- und Hydroxylgruppen, sind geeignet.

Im allgemeinen werden Olefine mit 2 bis 40, vorzugsweise mit 6 bis 20 Kohlenstoffatomen, eingesetzt, die nach bekannten Verfahren des Standes der Technik erhalten werden können. So können z. B. $\alpha$-Olefine durch Oligomerisierung von Ethylen nach Ziegler (DE-PS 878 560 und 1 190 930) oder durch Wachscracken, Olefine mit innenständiger Doppelbindung durch Dehydrierung oder Chlorierung und anschließender Dehydrochlorierung von Paraffinen (GB-PS 1 037 868) gewonnen werden.

Bei dem zuletzt genannten Verfahren werden in der Regel Paraffinschnitte, d. h. Gemische unterschiedlicher C-Zahl eingesetzt, so daß auch die erhaltenen Olefine keine einheitliche C-Zahl aufweisen.

Außerdem kommen in diesen Olefin-Gemischen natürlich alle denkbaren isomeren Formen vor. Neben den reinen — gegebenenfalls substituierten — Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die Art des Alkanols, das mit dem Olefin und Kohlenmonoxid umgesetzt wird, ist für das erfindungsgemäße Verfahren unkritisch. Im allgemeinen werden Alkanole mit 1 bis 10, vorzugsweise 1 bis 4 Kohlenstoffatomen, eingesetzt. Typische Vertreter aus der Gruppe der primären Alkanole sind z. B. Methanol, Ethanol, Propanol-(1) und Butanol-(1).

Weiterhin ist es unwesentlich, welche Kobaltverbindung bei der Hydrocarboxylierung verwendet wird. Carbonyle des Kobalts, z. B. Dikobaltoctacarbonyl, sind ebenso geeignet wie carbonsaure Kobaltsalze, wie z. B. Kobaltacetat, Kobaltnaphthenat und Kobalt-2-ethylhexanoat, und Salze des Kobalts mit anorganischen Säuren, wie z. B. Kobaltnitrat und Kobaltsulfat. Vorzugsweise kommen solche carbonsauren Kobaltsalze zum Einsatz, deren Anionen dem Säurerest der bei der Hydrocarboxylierung gebildeten Fettsäureester entsprechen.

Als Promotoren kommen Pyridin und alle nichtorthosubstituierten Alkylpyridine, wie z. B. 3- und 4-Picolin, 3,4- und 3,5-Lutidin und 3- und 4-Ethylpyridin bzw. Mischungen dieser Stoffe infrage.

Schließlich sind die Reaktionsbedingungen, unter denen die Hydrocarboxylierung durchgeführt wird, für das erfindungsgemäße Verfahren nicht von Bedeutung. Im allgemeinen werden Hydrocarboxylierungsverfahren bei Temperaturen von 80 bis 300, vorzugsweise 150 bis 220° C, und Kohlenmonoxiddrucken von 10 bis 800, vorzugsweise 100 bis 300 bar, durchgeführt.

Verfahrenskritisch für das vorliegende Verfahren ist jedoch die oxidative Behandlung des Reaktionsgemisches vor der Rückgewinnung des Kobalts mit Sauerstoff oder einem sauerstoffhaltigen Gas, vorzugsweise Luft, bei Temperaturen von 20 bis 150, vorzugsweise 40 bis 120° C. Diese Behandlung, die bereits in der US-PS 3 507 891, Spalte 4, Zeilen 21 bis 43, und in der deutschen Patentanmeldung P 29 12 489.8, entsprechend EP-A1-17 051, Seite 5, Absatz 2, beschrieben ist, wird bis zur Zerstörung der flüchtigen Kobaltverbindungen durchgeführt.

Entgegen den Angaben des Standes der Technik wurde nun gefunden, daß man durch die erfindungsgemäße Maßnahme eine so weitgehende Abtrennung aller nicht umgesetzten Einsatzstoffe und aller Reaktionsprodukte vom als Katalysator eingesetzten Kobalt erreicht, daß dabei keine Verdünnung des zurückzuführenden Kobalts durch Hochsieder auch bei beliebig häufiger Rückführung der gleichen Katalysatorcharge festgestellt wird.

Der Reaktionsaustrag, der unumgesetztes Alkanol und Olefin, Fettsäureester, den Promotor sowie Kobaltverbindungen als Katalysator als wesentliche Bestandteile enthält, wird in zwei Stufen in kobaltfreies Destillat und in ein das gesamte Kobalt enthaltende Konzentrat zerlegt. Man geht dabei so vor, daß man die niedriger siedenden Bestandteile zunächst in einer ersten Stufe abtrennt. Zu den niedriger siedenden Bestandteilen des Reaktionsgemisches zählen im allgemeinen Verbindungen, die bei Normaldruck noch mühelos verdampft werden können, wie z. B. niedere Alkanole mit 1 bis 5 C-Atomen, Olefine mit bis zu 8 C-Atomen sowie Pyridin.

In einer zweiten Destillationsstufe werden die höhersiedenden Bestandteile des Reaktionsgemisches zusammen mit den geringen während des Prozesses gebildeten Anteilen hochsiedender Nebenprodukte als Destillat abgetrennt. Im Destillat der 2. Stufe fallen im allgemeinen als wesentliche Bestandteile an: unumgesetztes Alkanol mit mehr als 5 Kohlenstoffatomen sowie Olefin mit mehr als 8 Kohlenstoffatomen, der als Reaktionsprodukt gebildete Ester sowie das als Promotor eingesetzte Pyridinderivat. Die Art der Aufteilung des Reaktionsaustrages in zwei Destillatschnitte wird im allgemeinen durch die individuelle Zusammensetzung des Reaktionsaustrages bestimmt. Bei entsprechender Zusammensetzung des Reaktionsgemisches sind selbstverständlich auch Verfahrensweisen möglich, die zwei Destillatfraktionen anderer als der oben angegebenen Zusammensetzung liefern.

Eine besonders häufig durch Hydrocarboxylierung zu lösende Aufgabenstellung ist die Herstellung

3

von Methylestern im Tensidbereich mit 11 bis 20 Kohlenstoffatomen der den Estern zugrunde liegenden Fettsäure unter Verwendung von Pyridin bzw. γ- oder β-Picolin als Promotor. Die sich in diesen Fällen stellende Trennaufgabe kann z. B. dadurch gelöst werden, daß in der 1. Destillationsstufe im wesentlichen Methanol und der 2. Destillationsstufe im wesentlichen der Promotor nicht umgesetztes Olefin sowie die als Reaktionsprodukt gebildeten Fettsäuremethylester als Destillat abgetrennt werden.

Die Trennoperationen des erfindungsgemäßen Verfahrens werden im allgemeinen in einer Kombination aus zwei Verdampfern durchgeführt. Wenn es die Siedepunkte der abzutrennenden niedrig siedenden Anteile des Reaktionsgemisches erlauben, wird die 1. Destillationsstufe vorzugsweise bei Normaldruck in einem Dünnschichtverdampfer oder einem anderen Verdampfer mit kurzer Verweilzeit des zu verdampfenden Produktes betrieben. Als besonders vorteilhaft hat sich für die Lösung der in der 1. Stufe durchzuführenden Trennaufgabe der Einsatz von Fallfilmverdampfern erwiesen, da auf diese Weise Verkrustungen des Apparates ganz oder weitgehend vermieden werden können. Die 2. Destillationsstufe wird zur Schonung des Produktes bei gegenüber der 1. Stufe vermindertem Druck betrieben. Ein störungsfreier Dauerbetrieb dieser 2. Stufe ist insbesondere beim Einsatz von Dünnschichtverdampfern als Destillationsapparat gewährleistet. Die Druckdifferenz bei den beiden Destillationsstufen ist im Prinzip unkritisch und wird im allgemeinen so eingestellt, daß die Destillate beider Stufen mit einfachen technischen Mitteln vollständig kondensiert werden können.

Unabhängig von der Wahl der Trennbedingungen der 2stufigen Destillation fällt der kobalthaltige Katalysator stets als Sumpf der 2. Destillationsstufe an. Durch die stete Ausschleusung von Hochsiedern unter den erfindungsgemäßen Bedingungen gelingt es, ohne daß Kobaltverluste auftreten, die Kobaltkonzentration dieses Sumpfes auch bei beliebig häufiger Wiederholung dieses Verfahrens konstant bzw. nahezu konstant zu halten.

Aus Gründen der Handhabbarkeit ist es zweckmäßig, den Destillationsrückstand nicht auf Kobaltgehalte über 20 Gewichtsprozent einzuengen. Kobaltgehalte unter 2 Gewichtsprozent im Destillationssumpf sind deshalb unzweckmäßig, da bei einer solchen Verfahrensweise ein unnötig großer Anteil des für die Hydrocarboxylierung zur Verfügung stehenden Reaktorvolumens durch die das Kobalt begleitenden Substanzen blockiert wird.

Innerhalb dieses Bereichs, vorzugsweise innerhalb des Bereichs von 4 bis 15 Gewichtsprozent Kobalt, läßt sich die Kobaltkonzentration beliebig durch entsprechende Wahl der Trennbedingungen in der 2. Destillationsstufe einstellen und — sofern erwünscht — über eine beliebige Zahl von Katalysatorkreisläufen konstant bzw. nahezu konstant halten.

Bei einer besonders bevorzugten Ausführungsform des erfindungsgemäßen Verfahrens, wählt man die Trennbedingungen der 2. Destillationsstufe so, daß als Sumpf ein kobalthaltiges Konzentrat anfällt, das bei 60°C eine Viskosität von $10^4$ bis $5 \cdot 10^5$ mPa s hat. Auf diese Weise ist gewährleistet, daß das Konzentrat mit den üblicherweise technisch zur Verfügung stehenden Pumpen problemlos gefördert werden kann.

Bevor der kobalthaltige Sumpf in die Hydrocarboxylierungsreaktion zurückgeführt wird, ist es erforderlich, ihn in dem als Promotor verwendeten Pyridin und/oder nichtorthosubstituierten Alkylpyridin aufzunehmen und die so erhaltene Suspension mit einem Gemisch aus Kohlenmonoxid und Wasserstoff bei einer Temperatur von 100 bis 250°C und einem Druck von mindestens 50 bar zu behandeln.

Zum Aufnehmen des kobalthaltigen Sumpfes wird vorzugsweise der aus dem Reaktionsaustrag wiedergewonnene Promotor benutzt, wobei eventuelle Verluste gegebenenfalls durch Zugabe frischen Promotors ausgeglichen werden.

Für die Behandlung der so erhaltenen Suspension mit dem Gemisch aus Kohlenmonoxid und Wasserstoff werden im allgemeinen Gasgemische mit 10 bis 90, vorzugsweise 40 bis 60 Volumenprozent Wasserstoff verwendet.

Die Behandlungstemperatur liegt zwischen 100 und 250°C, vorzugsweise zwischen 120 und 200°C; der Gesamtdruck muß mindestens 50 bar betragen. Eine obere kritische Grenze des Gesamtdruckes wird nicht durch den chemischen Ablauf des Verfahrens, sondern durch die Druckfestigkeit der zur Verfügung stehenden Apparate bestimmt. Bevorzugt arbeitet man im Druckbereich von 100 bis 400 bar.

Die Behandlungsdauer läßt sich leicht durch orientierende Versuche ermitteln. Sie richtet sich in erster Linie nach den gewählten Temperatur- und Druckbedingungen und sollte erfahrungsgemäß mindestens 5 Minuten betragen.

Der Erfolg der Behandlung läßt sich einmal daran erkennen, daß die in der eingesetzten Suspension enthaltenen Feststoffanteile in Lösung überführt werden, und zum anderen daran, daß die so erhaltene Katalysatorlösung wieder aktiv ist.

Die Behandlung der kobalthaltigen Suspension mit dem Gemisch aus Kohlenmonoxid und Wasserstoff kann z. B. in einem Druckgefäß, einer Kaskade von Druckgefäßen oder einem Rohrreaktor durchgeführt werden.

Das erfindungsgemäße Verfahren wird durch die nachfolgenden Beispiele näher erläutert.

### Beispiel 1

### A) Hydrocarboxylierung

Das Gemisch der Einsatzstoffe wird in folgenden Molverhältnissen

1 Mol n-Dodecen (Isomerengemisch mit einem Dodecen-(1)-Anteil $\leq$ 1 Gewichtsprozent)
2 Mol Methanol
0,3 Mol $\gamma$-Picolin
0,03 Grammatom Kobalt (in Form eines aus der Aufarbeitung nach C stammenden, das gesamte eingesetzte Kobalt enthaltenden Konzentrates, das nach D weiterbehandelt wurde)

kontinuierlich in einen Rühr-Autoklaven eingepumpt und dort unter den nachfolgenden Bedingungen zur Umsetzung gebracht:

| | |
|---|---|
| Reaktionstemperatur: | 185 °C |
| CO-Warmdruck | 180 bar |
| (CO enthält 1 Vol.-% H₂) | |
| Verweilzeit: | 1,6 h |

### B) Oxidation des Reaktionsaustrages

Der Reaktionsaustrag der Hydrocarboxylierungsstufe wird kontinuierlich einem mit 8 × 8 mm Raschigringen gefüllten, 1 m langen Rieselturm von oben zugeführt und mit Luft im Gegenstrom unter folgenden Bedingungen behandelt:

| | |
|---|---|
| Reaktionstemperatur: | 40 °C |
| Druck: | 1 bar |
| Flüssigkeitsdurchsatz: | 260 ml/cm² · h |
| Luft/1 l Reaktionsaustrag: | 50 l |

Das den Rieselturm verlassende Abgas wird durch Kondensation von organischen Inhaltsstoffen befreit. Diese werden dem oxidierten Reaktionsaustrag wieder zugeführt.

### C) Aufarbeitung des oxidierten Reaktionsaustrages

Der oxidierte Reaktionsaustrag wird kontinuierlich in einem Fallfilmverdampfer unter folgenden Bedingungen von unumgesetztem Methanol befreit:

— Normaldruck
— Heizmitteltemperatur = 197°C
— Temperatur der abfließenden Flüssigkeit = 166°C
— durch Einpumpen nicht verdampfter Flüssigkeitsanteile wird eine Flüssigkeitsbelastung eingestellt, die dem 10fachen der Zulaufmenge entspricht.

Das Sumpfprodukt des Fallfilmverdampfers wird kontinuierlich in einem Dünnschichtverdampfer soweit von flüchtigen Bestandteilen befreit, daß als Sumpf des Dünnschichtverdampfers (DSV) ein das gesamt Kobalt enthaltendes Konzentrat anfällt mit einem Kobaltgehalt von 8 Gewichtsprozent und mit einer Viskosität von $8,3 \cdot 10^4$ mPa s (bei 60°C). Bei dieser Trennoperation werden folgende Bedingungen eingehalten:

| | |
|---|---|
| — Druck am Übergang: | 40 mbar |
| — Heizmitteltemperatur: | 252 °C |
| — Umdrehungszahl des Rotors: | 900 U/min |

Die weitere destillative Aufarbeitung der in den ersten beiden Destillationsstufen (Fallfilmverdampfer, Dünnschichtverdampfer) erhaltenen Destillate liefert in 96% Ausbeute, bezogen auf umgesetztes Olefin (Umsatz 55%), ein Tridecansäuremethylestergemisch mit einem Anteil von 79% an linearem Ester.

### D) Behandlung des kobalthaltigen Konzentrates mit Kohlenmonoxid und Wasserstoff

Das als Sumpfprodukt des Dünnschichtverdampfers anfallende kobalthaltige Konzentrat wird mit dem aus der weiteren destillativen Aufarbeitung zurückgewonnenen $\gamma$-Picolin sowie mit der zum Ersatz der $\gamma$-Picolin-Verluste nötigen Menge an frischem $\gamma$-Picolin angemaischt und in einem Rühr-Autoklaven kontinuierlich unter folgenden Bedingungen mit einem Gemisch aus 50 Vol.-% CO und 50 Vol.- $H_2$ behandelt:

| Reaktionstemperatur: | 170 °C |
|---|---|
| Druck: | 200 bar |
| Verweilzeit: | 15 min |

Nach Entspannen auf 4 bar werden der Austrag dieser Stufe, frisches und aus der weiteren destillativen Aufarbeitung stammendes Methanol sowie frisches Olefin in den unter A angegebenen Molverhältnissen kontinuierlich der Hydrocarboxylierungsstufe zugeführt und erneut unter den in A genannten Bedingungen zur Umsetzung gebracht.

Bei insgesamt 100 Kreisführungen des Katalysators unter den in A, B, C und D beschriebenen Reaktions- bzw. Aufarbeitungsbedingungen wurden folgende, in Tabelle 1 aufgeführten Ergebnisse erzielt:

Tabelle 1

| Anzahl der Katalysator-kreisläufe | Olefin-umsatz [%] | Esterselek-tivität [%] | n-Anteil [%] | Co-Gehalt des DSV-Sumpfes [%] | Viskosität des DSV-Sumpfes [mPa s] | Gewicht des DSV-Sumpfes: Gewichts aller Einsatzstoffe außer CO |
|---|---|---|---|---|---|---|
| 1 | 55 | 96 | 79 | 8,0 | $8,3 \cdot 10^4$ | 0,08 |
| 10 | 53 | 97 | 80 | 7,9 | $7,0 \cdot 10^4$ | 0,08 |
| 25 | 56 | 96 | 79 | 8,1 | $9,0 \cdot 10^4$ | 0,08 |
| 50 | 54 | 95 | 78 | 7,8 | $6,2 \cdot 10^4$ | 0,08 |
| 75 | 57 | 96 | 78 | 8,0 | $8,7 \cdot 10^4$ | 0,08 |
| 100 | 55 | 96 | 80 | 8,1 | $9,5 \cdot 10^4$ | 0,08 |

Beispiel 2

Beispiel 1 wird wiederholt mit der Ausnahme, daß das zum Einsatz gelangende n-Dodecen-Isomerengemisch (Dodecen-(1)-Anteil ≤ 1 Gewichtsprozent), bezogen auf 1 Gewichtsteil Olefin, 0,29 Gewichtsteile nichtolefinischer Verbindungen enthält, die zusammen mit dem unumgesetzten Olefin bei der weiteren destillativen Aufarbeitung nach Beispiel 1C anfallen und die aus während der Hydrocarboxylierung gebildeten Nebenprodukten oder sich anreichernden Verunreinigungen des Ausgangsolefins bestehen.

Bei der weiteren destillativen Aufarbeitung nach Beispiel 1C des mit einem solchermaßen verunreinigten Olefin nach Beispiel 1A und B erhaltenen Reaktionsgemisches wird durch Ausschleusen eines Teils der Olefinfraktion der Anteil an nichtolefinischen Verunreinigungen konstant gehalten.

In der nachfolgenden Tabelle 2 sind die für 30 Kreisführungen des Katalysators erzielten Ergebnisse aufgeführt.

Tabelle 2

| Anzahl der Katalysator-kreisläufe | Olefin-umsatz [%] | Esterselek-tivität [%] | n-Anteil [%] | Co-Gehalt des DSV-Sumpfes [%] | Viskosität des DSV-Sumpfes [mPa s] | Gewicht des DSV-Sumpfes: Gewicht aller Einsatzstoffe außer CO |
|---|---|---|---|---|---|---|
| 1 | 51 | 95 | 80 | 7,9 | $9,5 \cdot 10^4$ | 0,07 |
| 5 | 50 | 96 | 81 | 7,8 | $10,1 \cdot 10^4$ | 0,07 |
| 10 | 49 | 97 | 79 | 8,0 | $9,2 \cdot 10^4$ | 0,07 |
| 20 | 50 | 96 | 80 | 8,1 | $9,5 \cdot 10^4$ | 0,07 |
| 30 | 50 | 96 | 80 | 8,0 | $8,9 \cdot 10^4$ | 0,07 |

Beispiel 3

Beispiel 1 wird wiederholt mit der Ausnahme, daß anstelle des n-Dodecen-Isomerengemisches Octen-(1) eingesetzt wird, folgende Molverhältnisse eingehalten werden:

| 1 | Mol Octen-1 |
|---|---|
| 3 | Mol Methanol |
| 0,45 | Mol $\gamma$-Picolin |
| 0,015 | Grammatom Kobalt |

und die Hydrocarboxylierungsreaktion unter folgenden Bedingungen durchgeführt wird:

| Reaktionstemperatur: | 165° C |
|---|---|
| CO-Warmdruck: | 270 bar |
| Verweilzeit: | 1,1 h |

In der nachfolgenden Tabelle 3 sind die für 30 Kreisführungen des Katalysators erzielten Ergebnisse aufgeführt.

Tabelle 3

| Anzahl der Katalysator-kreisläufe | Olefin-umsatz [%] | Esterselek-tivität [%] | n-Anteil [%] | Co-Gehalt des DSV-Sumpfes [%] | Viskosität des DSV-Sumpfes [mPa ] | Gewicht des DSV-Sumpfes: Gewicht aller Einsatzstoffe außer CO |
|---|---|---|---|---|---|---|
| 1 | 61 | 96 | 83 | 10,7 | $1,4 \cdot 10^5$ | 0,03 |
| 5 | 62 | 97 | 84 | 10,5 | $1,3 \cdot 10^5$ | 0,03 |
| 10 | 60 | 97 | 84 | 10,8 | $1,6 \cdot 10^5$ | 0,03 |
| 20 | 59 | 98 | 85 | 10,6 | $1,5 \cdot 10^5$ | 0,03 |
| 30 | 61 | 97 | 84 | 10,8 | $1,7 \cdot 10^5$ | 0,03 |

**Patentansprüche**

1. Verfahren zur Herstellung von Fettsäureestern durch Umsetzung von Olefinen, Alkanolen und Kohlenmonoxid in Gegenwart eines kobalthaltigen Katalysators und eines Promotors bestehend aus Pyridin und/oder nichtorthosubstituierten Alkylpyridinen, dadurch gekennzeichnet, daß man den Reaktionsaustrag, nach Behandlung bei Temperaturen von 20 bis 150° C mit einem sauerstoffhaltigen Gas, in 2 Destillationsstufen, von denen die erste bei höherem Druck als die zweite betrieben wird, schonend in kobaltfreies Destillat und ein als Sumpf der 2. Destillationsstufe anfallendes Konzentrat, das das gesamte als Katalysator eingesetzte Kobalt enthält, auftrennt, das kobalthaltige Konzentrat in Pyridin und/oder einem nichtorthosubstituierten Alkylpyridin aufnimmt, die so erhaltene Suspension mit einem Gemisch aus Kohlenmonoxid und Wasserstoff bei einer Temperatur von 100 bis 250°C und einem Druck von mindestens 50 bar behandelt und das Behandlungsprodukt in die Fettsäureestersynthese zurückführt.

2. Verfahren nach Anspruch 1, dadurch gekennzeichnet, daß das als Sumpf der 2. Destillationsstufe erhaltene Konzentrat zwischen 2 und 20 Gewichtsprozent Kobalt enthält.

3. Verfahren nach den Ansprüchen 1 und 2, dadurch gekennzeichnet, daß die Viskosität des als Sumpf der 2. Destillationsstufe erhaltenen kobalthaltigen Konzentrats bei 60°C $10^4$ bis $5 \cdot 10^5$ mPa s beträgt.

## Claims

1. A process for the production of a fatty acidester by reaction of an olefin, an alkanol and carbon monoxide in the presence of a cobalt-containing catalyst and a promotor selected from pyridine and alkylpyridines other than those substituted in the ortho-position, characterised in that the reaction mixture, after treatment at a temperature of 20 to 150°C with an oxygen-containing gas, is carefully separated in two distillation stages, the first of which is carried out at a higher pressure than the second, into a cobalt-free distillate and a concentrate which is formed as the bottoms product of the second distillation stage and contains all the cobalt added as catalyst, the cobalt-containing concentrate is taken up in pyridine and/or a non-ortho substituted alkylpyridine, the resulting suspension is treated at a temperature of 100 to 250°C and a pressure of at least 50 bar with a mixture of carbon monoxide and hydrogen and the product of the treatment is recycled to the fatty acid ester synthesis.

2. A process according to claim 1, characterised in that the concentrate obtained as the bottoms product of the second distillation stage contains from 1 to 20 wt.% cobalt.

3. A process according to claim 1 or 2, characterised in that the viscosity of the concentrate obtained as the bottoms product of the second distillation stage is from $10^4$ to $5 \times 10^5$ mPa s at 60°C.

## Revendications

1. Procédé de préparation d'esters d'acide gras par réaction d'oléfines, d'alcanols et de monoxyde de carbone en présence d'un catalyseur contenant du cobalt et d'un activeur formé de pyridine et/ou d'alkyl-pyridines non substituées en ortho, caractérisé par le fait qu'après avoir traité le produit de réaction à des températures de 20 à 250°C par un gaz oxygéné, on le sépare avec ménagement, en deux étapes de distillation dont on conduit la première à une plus haute pression que la deuxième, et distillat exempt de cobalt et en un concentré obtenu comme queues de la deuxième étape de distillation et qui contient la totalité du cobalt introduit comme catalyseur, que l'on reprend le concentré contenant du cobalt par la pyridine et/ou par une alkyl-pyridine non-substituée en ortho, que l'on traite la suspension obtenue par un mélange de monoxyde de carbone et d'hydrogène, à une température de 100 à 250°C et à une pression d'au moins 50 bar et que l'on ramène le produit de traitement à la synthèse d'esters d'acide gras.

2. Procédé selon la revendication 1, caractérisé par le fait que le concentré obtenu comme queues de la deuxième étape de distillation contient de 2 à 20% en poids de cobalt.

3. Procédé selon les revendications 1 et 2, caractérisé par le fait que la viscosité du concentré contenant du cobalt, obtenu comme queues de la deuxième étape de distillation, est, à 60°C, de $10^4$ à $5 \times 10^5$ mPa $\cdot$ s.